# EUROPEAN PATENT APPLICATION

(11) **EP 0 733 901 A2**
(43) Date of publication of application: **25.09.1996**
(21) Application number: 96104433.6
(22) Date of filing: 20.03.1996
(51) Int. Cl.: G01N 31/22, C07D 487/22

(54) **Gas reactive pigment, gas detector using the same, and gas detection method and apparatus using the detector**

(30) Priority: 20.03.1995 JP 85988/95; 18.10.1995 JP 270139/95; 07.03.1996 JP 50431/96
(71) Applicant: EBARA CORPORATION, Ohta-ku, Tokyo (JP); EBARA RESEARCH CO., LTD., Fujisawa-shi Kanagawa-ken (JP)
(72) Inventor: Tanaka, Kazunari, c/o Ebara Research Co., Ltd., Fujisawa-shi, Kanagawa-ken (JP); Igarashi, Chiaki, c/o Ebara Research Co., Ltd., Fujisawa-shi, Kanagawa-ken (JP); Sadaoka, Yoshihiko, Matsuyama-shi, Ehime-ken (JP)
(74) Representative: Hansen, Bernd, Dr. Dipl.-Chem.

(57) **Abstract**

A gas reactive pigment reactive to at least one of a halogen gas, a hydrogen halide gas, an acidic gas, an oxidizing gas, a basic gas, and an organic acid gas is provided. The pigment comprises a metal complex of tetraphenylporphyrin or a derivative thereof represented by formula (I) 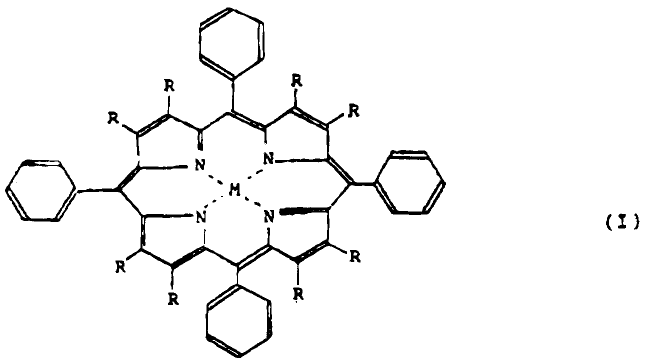 wherein M represents a transition metal; a plurality of R's independently represent a hydrogen atom, a halogen atom, a nitro group, a cyano group or an alkoxy group, or a tetraphenylporphyrin derivative represented by formula (II) 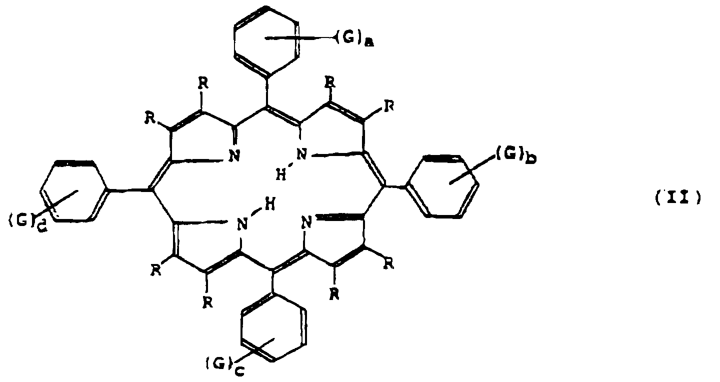 wherein a plurality of R's independently represent a hydrogen atom, a halogen atom, a nitro group, a cyano group or an alkoxy group; a plurality of G's on the 4 phenyl groups all represent a substituent selected from a halogen atom, a hydroxyl group, a sulfonic acid group, a carboxyl group, an alkoxy group, and an amino group, the positions of which on the 4 phenyl groups may be the same or different; and a, b, c and d which may be the same or different, each represents an integer of 0 to 5, indicating the number of G's on each phenyl group; provided that the compound wherein all R's represent a hydrogen atom, and a, b, c and d all represent 0 is excluded.

A detector for the above gases containing the reactive pigment; a method for detecting the above gases using the detector; and a detection apparatus for the above gases using the detector are also provided. The gas reactive pigment shows a color change, which can be measured in terms of the absorption spectrum change of said pigment at high sensitivity on contact with the above gases.

## Description

This invention relates to a pigment comprising a tetraphenylporphyrin (hereinafter referred to as TPP), a derivative thereof, or a metal complex thereof which has reactivity or reversible reactivity with a halogen gas, a hydrogen halide gas, an acidic gas, an oxidizing gas, a basic gas or an organic acid gas, and can be used for detecting these gases in a gas mixture which is suspected to contain at least one of these gases; and to a gas detector; a gas detection method; or a gas detecting apparatus using the detector comprising said pigment.

Various means for detecting harmful gases in gas mixtures such as air have hitherto been proposed. Unlike liquid components, gaseous components are relatively difficult to detect, and highly accurate measurement is difficult.

Detection means currently employed include (1) detection tubes, (2) color changing beads, (3) detection tapes, and (4) electrolysis at constant potential.

The detection tube (1) is a glass tube containing a detector comprising a carrier made of e.g., filter paper or silica gel. Provided on the carrier, is a substance which changes color upon reaction with a component to be detected (such as a pH test agent). The two ends of the tube are open to enable gases to come into contact with the detector. The detection tube can be used only once and hence requires to be changed. Detection sensitivity is in the order of ppm. In addition, it is impossible to conduct continuous measurement with the detection tube.

The detection beads (2) comprise beads of silica gel, etc. having supported thereon the above-mentioned detecting substance. They are placed at sites where the gas to be detected may exist. Their function is the same as the detection tube's. The detection beads can also be used only one time, though they are adaptable for continuous measurement.

The detection tape (3) comprises a carrier in the form of tape having provided thereon a detecting substance. The function and advantages are the same as those of the detection beads.

The constant potential electrolysis system (4) has a detection sensitivity in the order of ppm. Use of an electrolytic solution requires the trouble of replenishing the system with the electrolytic solution and correcting the sensitivity.

In addition, the color change in detectors (2) and (3) in response to harmful gases is irreversible. Therefore, after it has been constantly exposed to such a gas for a certain period even at a low concentration, it tends to erroneously change color reacting with the gas at a concentration lower than that to be detected. Further, because the gases to be detected are corrosive, the detector has poor durability.

It has recently been revealed that monophthalocyanine and certain "semi-conductor complex of cyclic compounds have properties of a gas detector. For example, PCT WO 91/07658 (unexamined Japanese Patent Publication No. Hei 5-505456) discloses that metallo-bisphthalocyanine pigments, bistetrabenzoporphyrin, and bis(mono-, di- and tri-)azatetrabenzoporphyrins or mixtures thereof serve as gas detecting components of a gas sensor. However, this disclosure is limited to the spectrum change of tetrabenzoazaporphyrin bis-macrocyclic compounds upon exposure to gaseous chlorine.

PCT WO 91/07659 (Unexamined Japanese Patent Publication No. Hei 5-505871) discloses a gas sensor comprising mono-, (di- or tri-)azatetrabenzoporphyrin as the gas detecting component, which changes its color or electric conductivity when exposed to certain kinds of gases. The porphyrin compounds contain chromium (III), vanadium, manganese, cobalt or iron (III).

The above gas sensors are capable of detecting a gas component by virtue of a change in color or conductivity which takes place upon exposure to a certain gas component, but their sensitivity is not particularly high. It is noted that some of the gas detecting components exhibit restoration of their gas detecting ability to some extent, but it generally takes a long time for the sensor to restore the ability. Of the gas sensors disclosed, even that which requires the shortest time for restoration is not at all sufficient for continuous measurement.

Further, the degree of color change in these conventional substances is not sufficient to provide the sensitivity required for practical use. Of these substances the triazatetrabenzoporphyrin, whose sensitivity is not so high, is represented by the following formula:

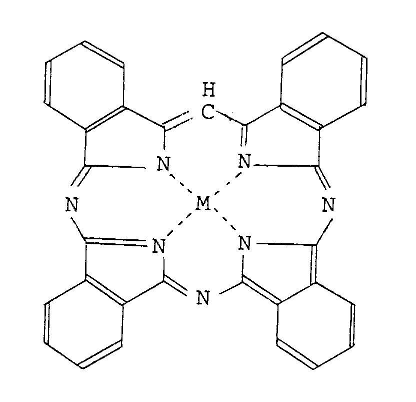

BESWICK et al. have reported that fluorescence was emitted from the TPP/AA Langmuir-Blodgett (LB) membrane, prepared from arachidic acid (AA) and TPP, upon excitation with the wavelength of 440 nm, and that said fluorescence was quenched on contact with nitrogen oxide, hydrogen chloride or chlorine gas (see J. Coll. Sci. and Eng., Vol. 124, p. 146 (1988)).

There has been strong demand for development of a detection system enabling rapid, simple, and highly accurate detection of harmful components in a gas mixture such as air. It would be of great benefit to environmental conservation to detect harmful components in exhaust gas discharged, e.g., from semiconductor production systems, etc., such as halogen or hydrogen halide, rapidly, easily and accurately.

While detection of SOₓ or NOₓ has conventionally been of interest, halogen and hydrogen halide gases have now become a more serious problem in the field of semiconductor production. Therefore, it is desired to develop a means by which halogen or halide gases as well as SOₓ and NOₓ can be detected with both ease and accuracy.

An object of the present invention is to provide a gas reactive pigment which undergoes changes measurable in terms of spectrum changes on reaction with a halogen gas, a hydrogen halide gas, an acidic gas, an oxidising gas, a basic gas or an organic acid gas and is therefore useful for detecting such harmful gases, especially a halogen gas or a hydrogen halide gas, in gas mixtures containing them.

Another object of the present invention is to provide a gas detector which is useful for detecting harmful components, such as a halogen gas and a hydrogen halide gas, in gas mixtures containing them.

A further object of the present invention is to provide a detection method and a detection apparatus using a gas detector for detecting harmful components, such as a halogen gas and a hydrogen halide gas, in gas mixtures containing them.

Fig. 1 shows an example of the reflection type gas detection apparatus according to the present invention.

Fig. 2 shows an example of the optical fiber type gas detection apparatus according to the present invention.

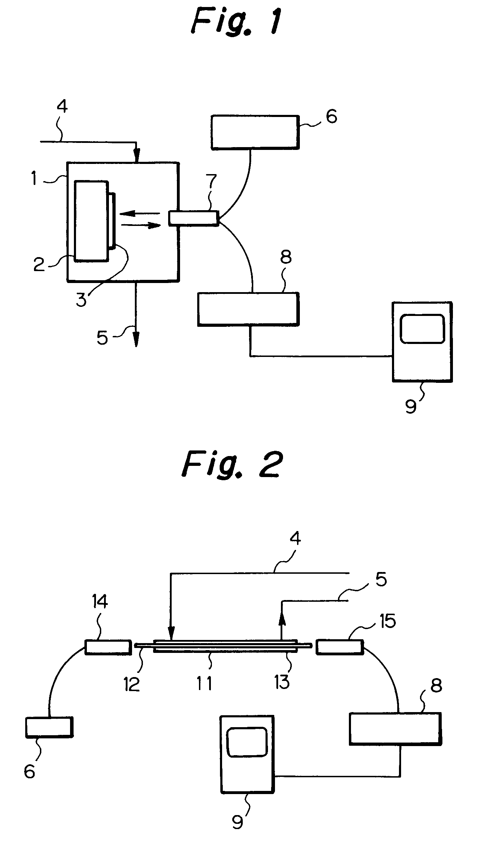

The reference numerals in Figs. 1 and 2 indicate the following members:
- 1 ...: Measuring cell 2 ... Substrate
- 3 ...: Detector 4 ... Gas to be examined
- 5 ...: Exhaust gas 6 ... Halogen lamp
- 7 ...: Projector and receptor
- 8 ...: Multiphotometry detection instrument
- 9 ...: Computer
- 11 ...: Measuring cell 12 ... Optical fiber
- 13 ...: Passage 14 ... Projector
- 15 ...: Receptor

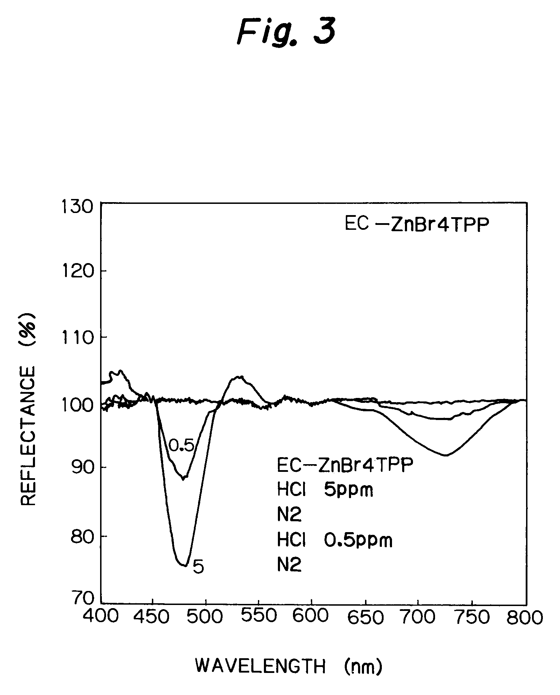

Fig. 3 is a graph showing the change in reflectance of one of the reactive pigments of the invention vs. wavelength, in the reaction with hydrogen chloride-containing gas.

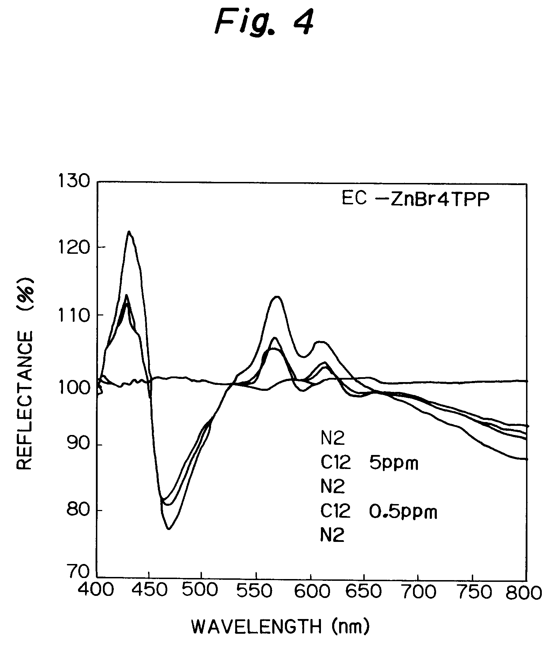

Fig. 4 is a graph showing the change in reflectance of one of the reactive pigments of the invention vs. wavelength, in the reaction with chlorine-containing gas.

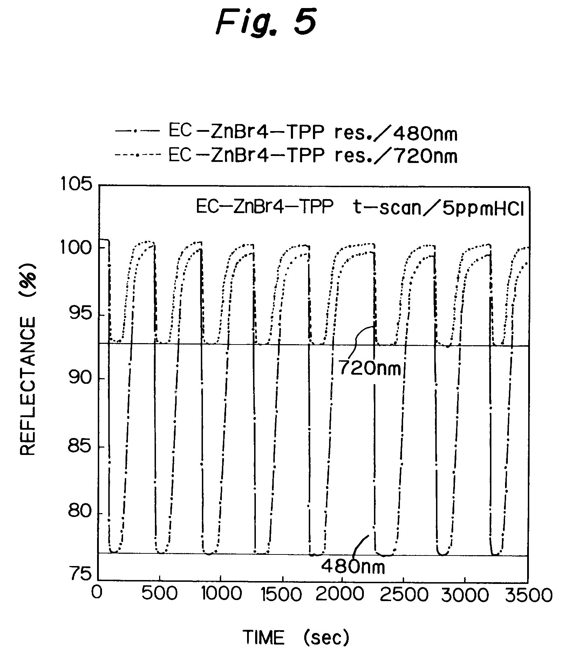

Fig. 5 is a graph showing the response characteristics of one of the reactive pigments of the invention, measured at two wavelengths, in the reaction with hydrogen chloride-containing nitrogen gas.

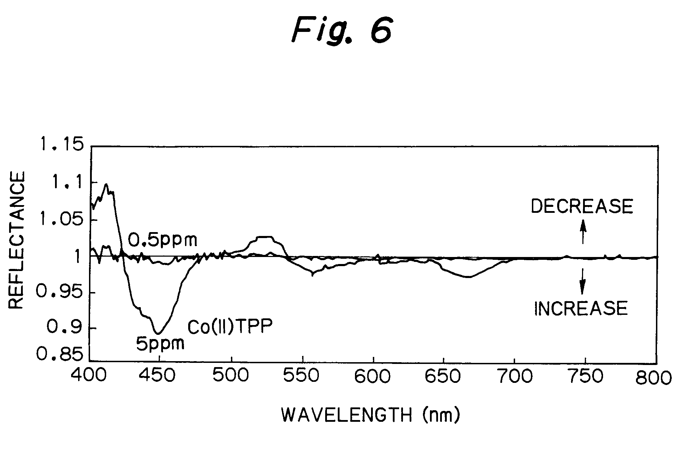

Fig. 6 is a graph showing the change in reflectance of another reactive pigment of the invention vs. wavelength, in the reaction with hydrogen chloride-containing nitrogen gas.

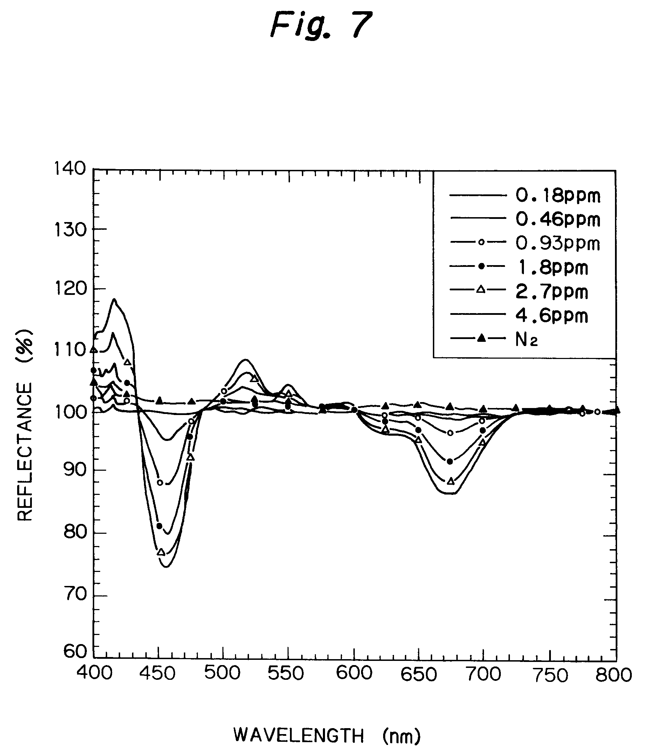

Fig. 7 is a graph showing the change in reflectance of 5,10,15,20-tetrakis(4-bromophenyl)porphyrin vs. wavelength, in the reaction with hydrogen chloride-containing nitrogen gas.

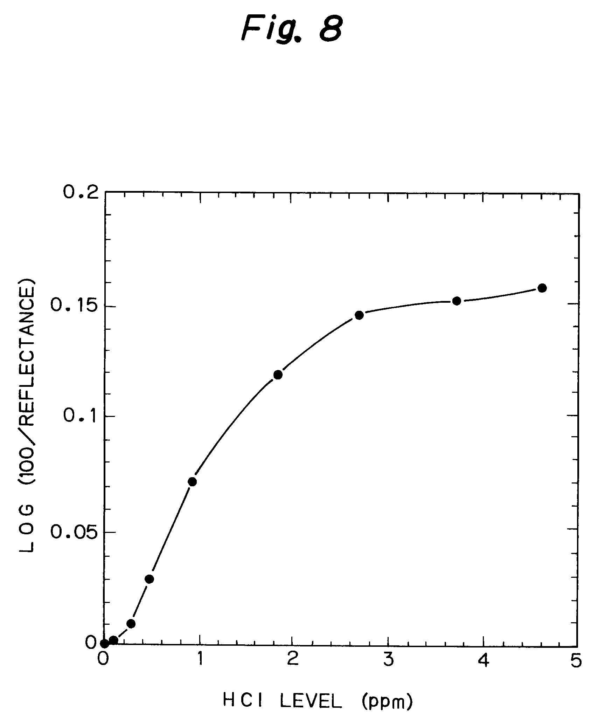

Fig. 8 is a graph of log(100/reflectance) vs. hydrogen chloride gas concentration, in the reaction of 5,10,15,20-tetrakis(4-bromophenyl)porphyrin with hydrogen chloride-containing nitrogen gas.

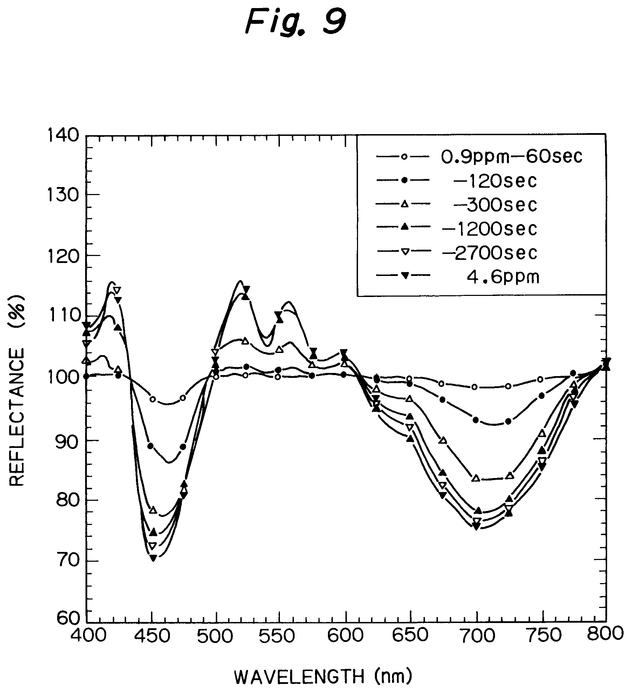

Fig. 9 is a graph showing the change in reflectance of 5,10,15,20-tetrakis(4-hydroxyphenyl)porphyrin vs. wavelength, in the reaction with hydrogen chloride-containing nitrogen gas.

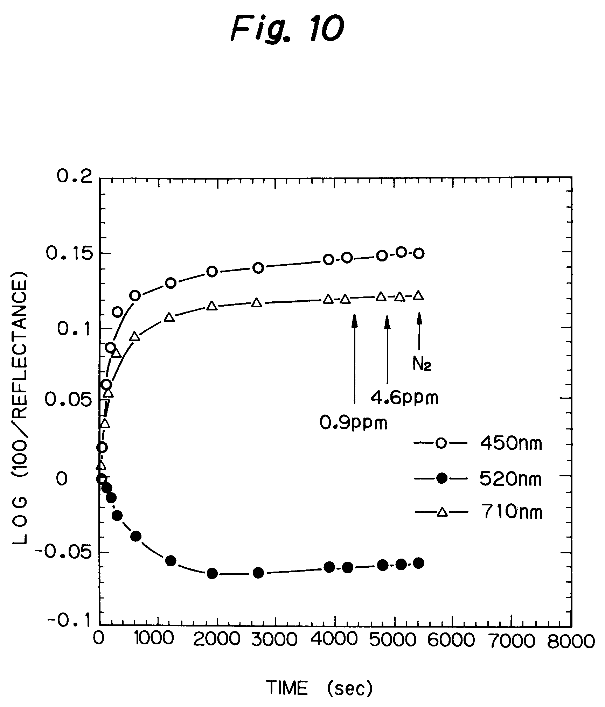

Fig. 10 is a graph showing the change in response in terms of log(100/reflectance) with time, in the reaction of 5,10,15,20-tetrakis(4-hydroxyphenyl)porphyrin with hydrogen chloride-containing nitrogen gas.

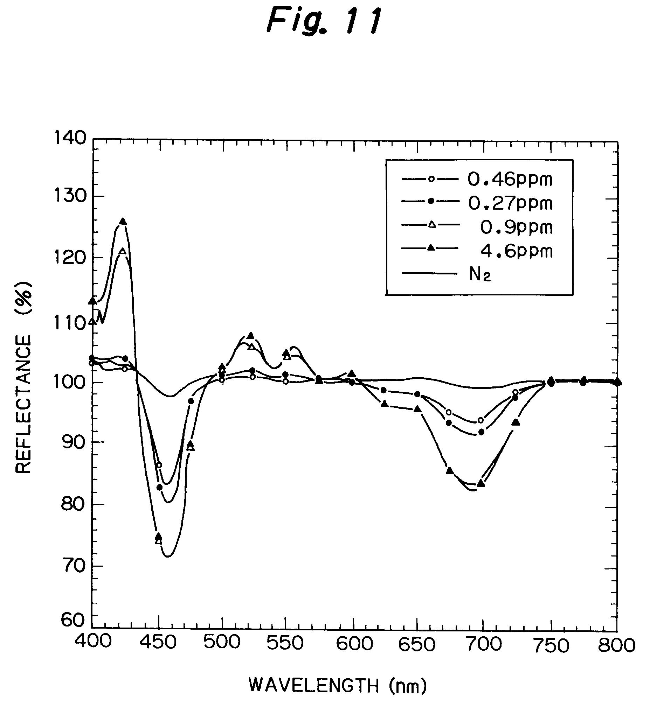

Fig. 11 is a graph showing the change in reflectance of 5,10,15,20-tetrakis(4-methoxyphenyl)porphyrin vs. wavelength, in the reaction with hydrogen chloride-containing nitrogen gas.

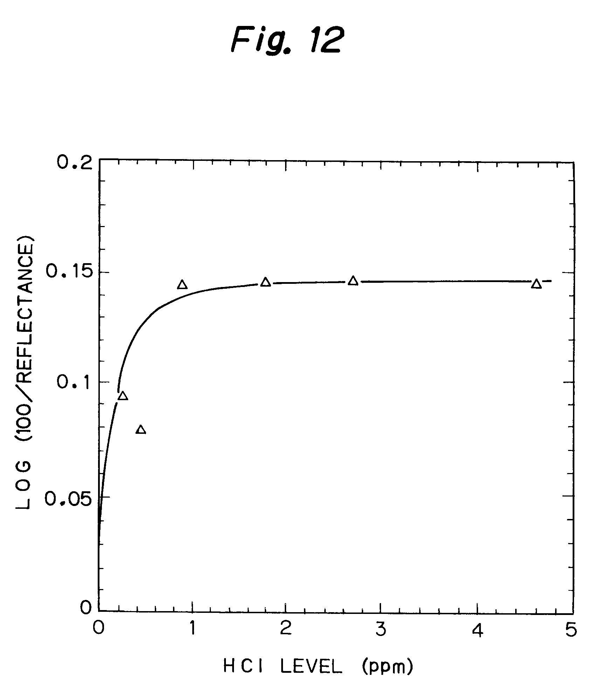

Fig. 12 is a graph of log(100/reflectance) vs. hydrogen chloride gas concentration, in the reaction of 5,10,15,20-tetrakis(4-methoxyphenyl)porphyrin with hydrogen chloride-containing nitrogen gas.

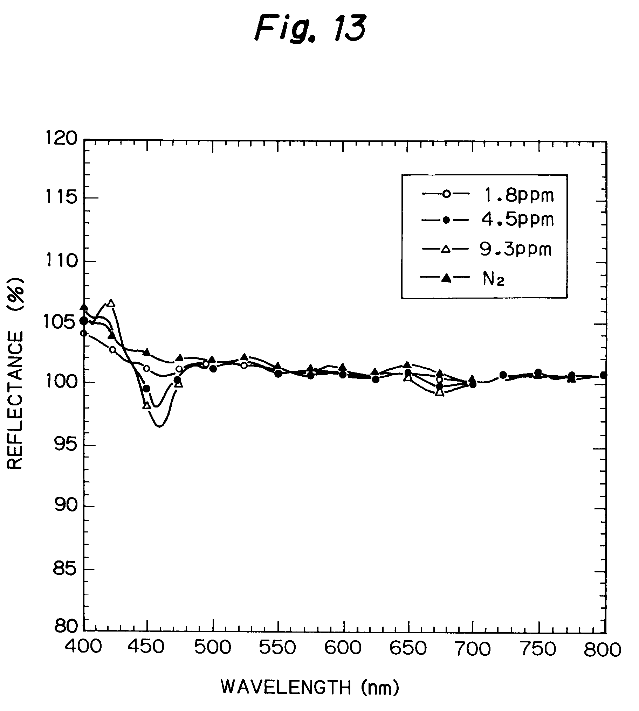

Fig. 13 is a graph showing the change in reflectance of tetrakis(4-carboxyphenyl)porphyrin vs. wavelength, in the reaction with hydrogen chloride-containing nitrogen gas.

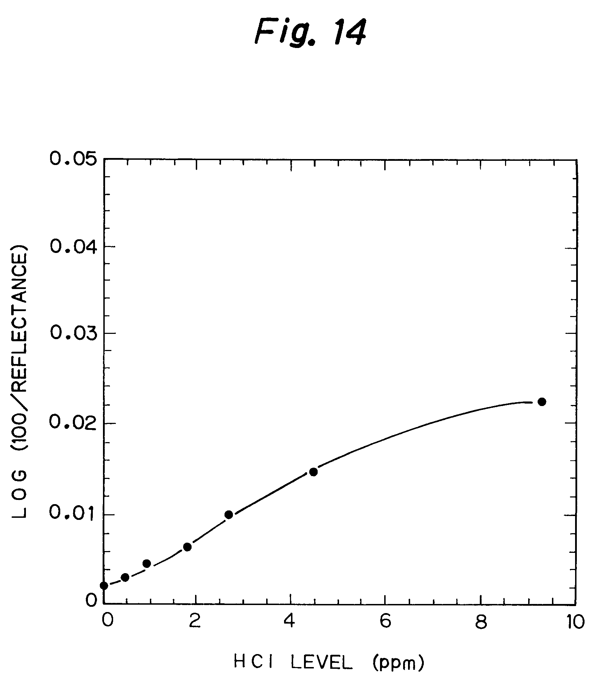

Fig. 14 is a graph of log(100/reflectance) vs. hydrogen chloride gas concentration, in the reaction of tetrakis(4-carboxyphenyl)porphyrin with hydrogen chloride-containing nitrogen gas.

In one aspect of the present invention, there is provided a gas reactive pigment comprising a metal complex of TPP or a TPP derivative represented by formula (I): 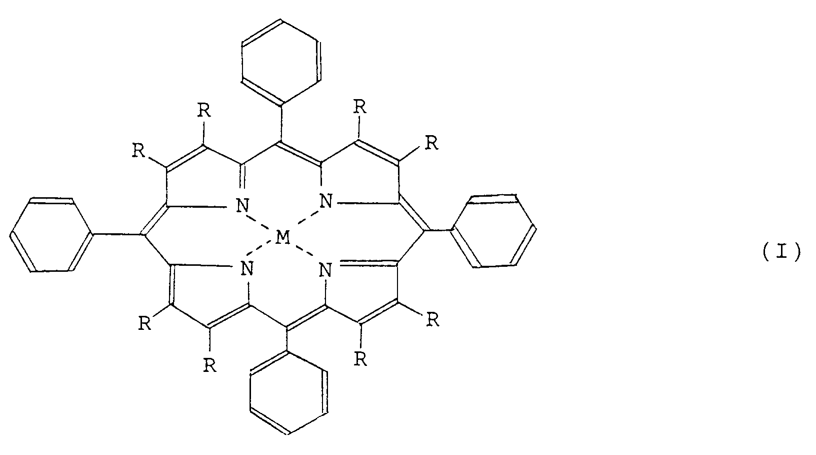 wherein M represents a transition metal; and R represents a hydrogen atom, a halogen atom (i.e., fluorine, chlorine, bromine or iodine), a nitro group, a cyano group or an alkoxy group (preferably an alkoxy group having 1 to 3 carbon atoms),
wherein the pigment has reactivity to at least one of a halogen gas, a hydrogen halide gas, an acidic gas, an oxidizing gas, a basic gas, and an organic acid gas (hereinafter inclusively referred to as harmful gas(es)).

The present invention preferably provides:
a gas reactive pigment represented by formula (I) (hereinafter referred to as gas reactive pigment (I)) wherein R is a bromine atom;
a gas reactive pigment (I) wherein M is zinc;
a gas reactive pigment (I) wherein M is zinc, and R is a bromine atom;
a gas reactive pigment (I) wherein M is zinc, and 3 to 5 or even more out of 8 R's are bromine atoms; and
a gas reactive pigment (I) wherein M is iron, and each of all R's is a bromine atom.

In another aspect, the present invention provides a gas reactive pigment comprising a TPP derivative represented by formula (II): 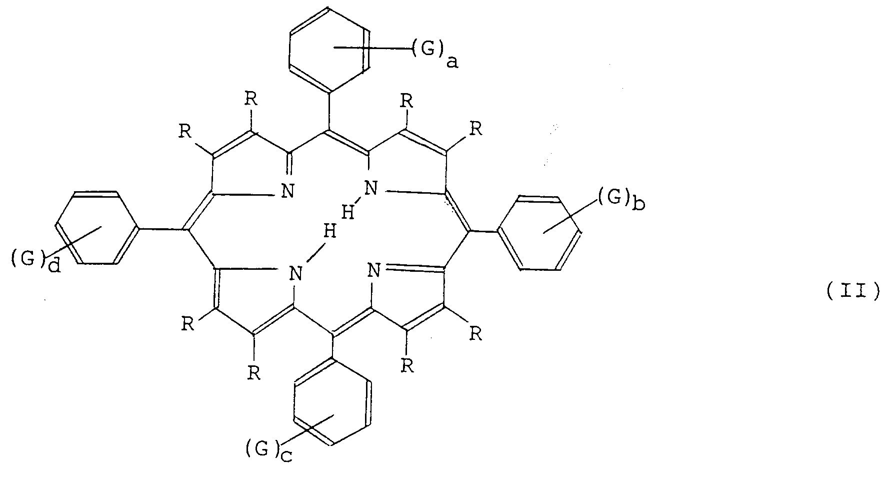 wherein R represents a hydrogen atom, a halogen atom (i.e., fluorine, chlorine, bromine or iodine), a nitro group, a cyano group or an alkoxy group (preferably an alkoxy group having 1 to 3 carbon atoms); the plurality of G's on the 4 phenyl groups independently represent a substituent selected from a halogen atom, a hydroxyl group, a sulfonic acid group, a carboxyl group, an alkoxy group, and an amino group, the positions of which on the phenyl groups may be the same or different; and a, b, c, and d, which may be the same or different, each represents an integer of 0 to 5, indicating the number of G's on each phenyl group; provided that the compound wherein all R's represent a hydrogen atom, and a, b, c, and d all represent 0 is excluded, wherein the pigment has reactivity to at least one of the above mentioned harmful gases.

The present invention preferably provides a gas reactive pigment represented by formula (II) (hereinafter referred to as gas reactive pigment (II)) wherein the G's independently represent one of a hydroxyl group, a carboxyl group, a sulfonic acid group, a bromine atom, and a methoxy group.

In still another aspect, the invention provides a gas detector for detecting at least one of the above mentioned harmful gases, which comprises the gas reactive pigment (I) or (II).

The present invention preferably provides a gas detector comprising a carrier having the gas reactive pigment provided thereon.

The present invention preferably provides a gas detector prepared by the steps of: dissolving the gas reactive pigment in a solvent together with a matrix polymer; applying the solution to the surface of a carrier; and drying the solution to remove the solvent.

The present invention preferably provides a gas detector in which the matrix polymer is a polymer soluble in a solvent for the gas reactive pigment, and the carrier is an alumina substrate, a glass substrate, an optical fiber, paper, glass beads or silica gel.

In a further aspect, the present invention provides a gas detector comprising Ethyl Violet in combination with the gas reactive pigment.

In a further aspect, the invention provides a method for detecting at least one of the above-mentioned harmful gases, which comprises bringing the gas to be examined into contact with any one of the above-mentioned gas detectors and measuring color change of the gas reactive pigment.

The invention preferably provides a method for detecting harmful gases, in which the color change of the gas reactive pigment is measured on the basis of the change in the absorption or reflective spectrum of the gas reactive pigment in the detector.

The invention preferably provides a method for detecting harmful gases, in which the change in the absorption or reflective spectrum of the reactive pigment is measured at a specific wavelength of the spectrum, and the measurement is conducted with regard to light which is transmitted through or reflected from the detector while the detector is being irradiated with a light source. This embodiment may be modified such that the gas reactive pigment is excited with a light source at a specific wavelength, and intensity of the fluorescence emitted from the pigment is measured. In the presence of a harmful gas, the pigment will change in terms of the fluorescence level or the wavelength at which it is excited or it emits fluorescence.

The invention preferably provides a method for detecting harmful gases, in which the change in the absorption or reflective spectrum of the reactive pigment is measured at a plurality of wavelengths of the spectrum, and the measurement is conducted with regard to light which is transmitted through or reflected from the detector while the detector is being irradiated with a light source. In this respect, the changes in absorbancy at the plurality of wavelengths will differ. Such differences can be used to enhance the sensitivity of the gas detection.

In a still further aspect, the invention preferably provides a method for detecting at least one of the above mentioned harmful gases, which comprises bringing the gas to be examined into contact with a gas detector containing the gas reactive pigment TPP of formula (III): 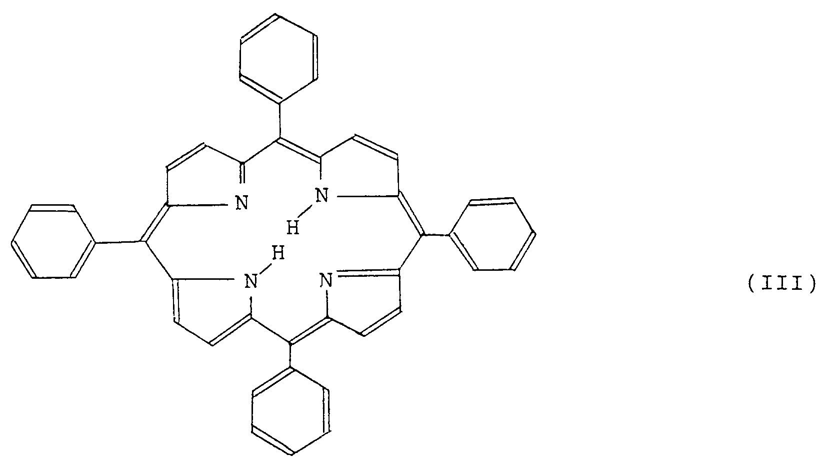 and measuring the color change of the gas reactive pigment, while the detector is being irradiated with light, on the basis of the changes in the absorption or reflective spectrum at one or more specific wavelengths, wherein the measurement is performed with regard to light which is transmitted through or reflected from the detector.

In a yet further aspect, the present invention provides a gas detection apparatus for detecting at least one of the above mentioned harmful gases, which has one of the above-mentioned gas detectors placed in a passage where gas to be examined flows through.

### Gas Reactive Pigment

In formula (I) representing the metal complex of TPP or a derivative thereof, the transition metal M includes zinc, copper, iron, nickel, cobalt, manganese, titanium, and aluminum. Zinc, cobalt, and iron are preferred. R represents a hydrogen atom, a halogen atom (fluorine, chlorine, bromine or iodine), a nitro group, a cyano group or an alkoxy group (e.g., methoxy or ethoxy). A preferred halogen atom is bromine. The phenyl groups may have one or more substituents as long as they do not alter the gas reactivity of the pigment.

The gas reactive pigment (I) changes color on contact with the above-described harmful gases: the color change is so significant that it affords high sensitivity to the detection of harmful gases. The initial color of the pigment and the color after reaction depend on the transition metal M contained therein.

The color change of the reactive pigment (I) is generally reversible. That is, the reactive pigment (I) reverts to its original color if the gas is removed after reaction with said gas. Reversibility varies depending on the transition metal and also on the gas to be reacted. For example, the reactive pigments (I) containing zinc as the transition metal reversibly react with hydrogen chloride gas, showing rapid and reversible coloration and restoration, enabling continuous measurement.

Those TPP derivatives of the present invention which are not metal complexes are also effective as gas reactive pigments for gas detection. That is, the reactive pigment comprising the TPP derivative represented by formula (II) (reactive pigment (II)) is also excellent in that it shows significant color change upon reaction with gas. Of the reactive pigments (I) and (II), preferred are those in which R is a bromine atom. The number of bromine atoms as R per molecule can be from 0 to 8. When reactive pigments (I) and (II) have no or smaller number of bromine atoms as R per molecule they are preferred for their ability to revert to their original color. As the number becomes larger, the pigments are reactive with harmful gases at lower levels.

With regard to the gas reactive pigment of formula (II), if the substituents G on the 4 phenyl groups bonded to the porphyrin skeleton are taken as (G₁)ₐ, (G₂)_{b}, (G₃)_{c}, and (G₄)_{d}, it is preferred that they have the same meaning (G₁=G₂=G₃=G₄). The suffixes a, b, c, and d, which denote the number of the substituents G on each phenyl group, may be the same or different and represent an integer of 0 to 5. The position of G on the phenyl group may be the same or different among the 4 phenyl groups.

Specific examples of G include a halogen atom, a hydroxyl group, a sulfonic acid group, a carboxyl group, an alkoxy group (e.g., methoxy or ethoxy), and an amino group. Preferred of them are a hydroxyl group, a carboxyl group, a sulfonic acid group, a bromine atom, and a methoxy group.

In the TPP or derivatives thereof of formula (II), where G represents one of the above-mentioned substituents, R preferably represents a hydrogen atom.

The pigments of the present invention are generally known compounds. If necessary, tetraphenylporphyrin (chlorine free); 5,10,15,20-tetrakis(4-hydroxyphenyl)-21H,23H-porphyrin; 5,10,15,20-tetrakis(4-methoxyphenyl)-21H,23H-porphyrin; 5,10,15,20-tetrakis(4-bromophenyl)-21H,23H-porphyrin; and tetraphenylporphyrin tetrasulfonic acid are commercially available from Tokyo Kasei Kogyo Co., Ltd., Tokyo Japan. If some of the pigments are not commercially available, they can be prepared in accordance with known methods. For example, for the preparation of metalloporphyrins, refer to J. W. Buchler, "Porphyrins and Metalloporphyrins", ed. by K. M. Smith, p. 157, Elsevier, New York (1975); for the preparation of tetraphenylporphyrin s having one or more substituents at positions 1 to 8, refer to Francis, D., Anne, V., Eric, V.C., Michelle, F., Tristano, B., Pietro, T., Karl, M.K.J., Inorg. Chem., 1993, 32, 4042 and Giraudeau, A., Callot, H. J., Gross, M., Inorg. Chem., 1979, 18, 201.

### Combination with Ethyl Violet

The reactive pigment (I), (II) or (III) can be used in combination with Ethyl Violet. Thus, the color change will be enhanced to provide higher detection sensitivity. The mechanism of the color change enhancement due to the combined use of Ethyl violet is not clear, but it is speculated that the amine moieties in Ethyl Violet molecule interact with porphyrin ring thereby to enhance the reactivity of the pigment.

### Gas Detector

The gas reactive pigments of the invention change the electronic state in the molecule thereof upon reaction with gases having high proton-donating properties or high proton-accepting properties, such as a halogen gas, a hydrogen halide gas, an acidic gas, an oxidizing gas, a basic gas and an organic acid gas. The gas reactive pigment can therefore be used to provide a detector for detection of these gases. For example, a gas detector can be prepared by dissolving the gas reactive pigment in an appropriate solvent together with a matrix polymer, applying the pigment solution to a carrier, and drying the solution to remove the solvent. When a gas, preferably a gas having high proton-donating properties or high proton-accepting properties, is brought into contact with such a detector, the reactive pigment supported on the carrier undergoes a color change, which can be measured in terms of the spectrum change in the light applied to the detector and transmitted therethrough or reflected therefrom. As mentioned above, gases detectable with the detector of the invention include those having high proton-donating or accepting properties. The term "measurement of the spectrum change in the reactive pigment" will hereinafter be used to refer to the measurement of the color change in the reactive pigment provided on the carrier, by way of the spectrum change in the light transmitted through or reflected from the detector.

The gases detectable by the present invention are as mentioned above. The basic gases include ammonia gas. The organic acid gases include formic acid gas. The acidic gases include HCl, HF, HBr, etc. The oxidizing gases include Cl₂, F₂, Br₂, etc. Detection or measurement of these harmful gases is required, for example, in semiconductor production plants.

The color change of the gas reactive pigment which takes place upon contact with the specific gas, can be measured by way of a change in the light absorption spectrum. The light source to be used for measurement of the spectrum change in the transmitted or reflected light includes natural white light, a halogen lamp, or one or more monochromatic light beams.

Since the gas reactive pigments of the invention exhibit significant changes in their colors upon contact with harmful gases, and said changes can be detected by way of changes in their light absorption spectrum peaks, the gas detectors of the invention have high detection sensitivity.

The colors of the reactive pigment, i.e., the original color before contact with a harmful gas and the color after the contact as measured by the peak wavelength and peak intensity will vary depending on the individual gas reactive pigment.

Also, the reversible changes in respect of peak wavelength and peak intensity in their spectra, exhibited by the gas reactive pigments of the invention, depend on the gas to be reacted. For example, the gas reactive pigment used in detector No. 2 of Example 1 hereinafter described exhibits satisfactory reversibility in the reaction with hydrogen chloride gas, as observed by the excellent reversibility in the peak wavelength and the intensity of the spectrum. Further, this pigment quickly reverts to its original state. Therefore, it is suitable for use in continuous measurement. Additionally, this pigment is reactive to such a low concentration of chlorine gas as about 0.5 ppm with considerable reversibility. Further, the pigments used in detector Nos. 7 and 8 of Example 4 hereinafter described also show substantial sensitivity to about 1 ppm of hydrogen chloride gas or about 10 ppm of NOₓ with excellent reversibility and are therefore of high practical utility.

### Production of Gas Detector

In order to make the gas reactive pigment suitable for use in gas detection, it is recommended that the pigment be mixed with at least a semi-transparent polymer matrix, preferably a transparent polymer matrix and that the mixture be applied on a carrier to provide a detector. This enables one to measure the change in the absorption spectrum of the gas reactive pigment with ease and makes it easier for the pigment to undergo reaction with harmful gases and thereby enhance sensitivity.

For example, it is preferable for the reactive pigment to be dissolved in a solution of a high polymer that is transparent and has no absorption peaks which may interfere with the absorption spectrum of the pigment, such as ethyl cellulose, and that the solution is applied to a carrier, such as an alumina substrate, a glass substrate, an optical fiber, and the like. The use of a carrier facilitates the measurement.

The matrix polymer which can be used for supporting the gas reactive pigment on a carrier is not particularly limited as long as it is sufficiently transparent for the purpose of the invention and has substantially no adsorption peak in the absorption wavelength region of the gas reactive pigment.

Examples of such polymers include Nafion (a registered trade mark), ethyl cellulose, ethoxyethyl cellulose, cellulose acetate, cellulose acetate butyrate, modified starch, polyvinylpyrrolidone, vinylpyrrolidone copolymers, polymethyl vinyl ether, and methyl vinyl ether copolymers.

Non-limiting examples of preferred carriers on which the mixture of the polymer and the gas reactive pigment is supported include an alumina substrate, a glass substrate, an optical fiber, paper, glass beads, and silica gel.

It has been observed that the spectrum change of the gas reactive pigment upon reaction with a halogen gas or a hydrogen halide gas does not take place uniformly throughout the wavelengths of the spectrum. Comparison of the reflective spectra before and after the reaction reveals that, in general, appreciable changes take place at specific wavelengths.

For example, Fig. 7 shows the results of an experiment using a detector comprising ethyl cellulose as a polymer matrix and 5,10,15,20-tetrakis(4-bromophenyl)porphyrin (i.e., the compound (II) wherein R is H, G=Br, and each of a, b, c and d=1; hereinafter referred to as TP(Br)P) as the gas reactive pigment. In this experiment, the detector was brought into contact with N₂ gas containing HCl in a concentration of 0.18 ppm, 0.46 ppm, 0.93 ppm, 1.8 ppm, 2.7 ppm or 4.6 ppm, or N₂ gas containing no HCl (background). The reflectance of the detector was measured at varied wavelengths, and the results obtained were plotted taking the reflectance of the background as a base line (100%).

It can be seen from the results that harmful gases can be detected with the detector of the invention, and that improved sensitivity can be assured by using a light beam having a specific wavelength at which the detector exhibits a large change in its reflective spectrum. The results also show that two or more beams having different wavelengths may be used for the measurement. Further, a wavelength at which the reflective spectrum does not change can also be used in combination. In that case, the results obtained at that wavelength provides a base line, making it possible to carry out continuous measurement without using a separately determined base line.

### Detection Apparatus

The detection apparatus using the detector comprising the reactive pigment of the invention may comprise the detection system as exemplified in Fig. 1, in which a detector comprising alumina substrate 2 coated with the gas reactive pigment is irradiated vertically with a light beam from a halogen lamp 6, and the reflected light is measured with multiphotometry detection instrument 8.

Another example of the detection apparatus is shown in Fig. 2, in which a detector comprising optical fiber 12 coated with the gas reactive pigment is placed in a gas passage where hydrogen chloride-containing gas, for example, is passed through. A light beam from halogen lamp 6 is led to optical fiber 12 by way of projector 14, and the beam from optical fiber 12 is received by receptor 15 and measured in multiphotometry detection instrument 8.

### Detection of Harmful Gas

The reactive pigment of the invention changes color upon reaction with harmful gases such as a halogen gas, a hydrogen halide gas, an acidic gas, an oxidizing gas, a basic gas or an organic acid gas, and thus detects the presence of these specific gases. It has not been known that the gas reactive pigments (I) and (II) have such properties.

The gas detector of the invention, which has the gas reactive pigment mixed in a transparent matrix or supported on a transparent carrier, can be conveniently used in the detection of the spectrum change of the pigment.

The detection of the spectrum change is preferably carried out using a light beam having a wavelength at which the reactive pigment shows its maximum change in the absorption spectrum thereof.

The color change of the reactive pigment upon reaction with halogen, hydrogen halide, NOₓ or SOₓ does not occur uniformly throughout the entire wavelengths of the spectrum. The applicants have found that remarkable changes of the reflective spectra before and after the reaction are observed, in general, at specific wavelengths. For example, Fig. 3 shows the results of the experiment using a detector comprising ethyl cellulose as the polymer matrix and the tetraphenyltetrabromoporphyrin of formula (I) wherein M is a zinc atom and R is a bromine atom as the gas reactive pigment (the pigment will hereinafter be referred to as ZnBr4-TPP, and the detector will hereinafter be referred to as EC/ZnBr4-TPP), in which the detector was brought into contact with N₂ gas containing HCl in a concentration of 0.5 ppm or 5 ppm or N₂ gas containing no HCl (background), the reflectance of the detector was measured at a varied wavelength, and the results obtained were plotted taking the reflectance of the background as a base line (100%).

As the results in Fig. 3 show, in detecting a specific gas with the detector of the invention, improved sensitivity is warranted by using a light beam having a specific wavelength at which the detector exhibits a large change in the reflectance thereof. Two or more beams having different wavelengths may be used for the measurement. Further, a wavelength at which the reflective spectrum does not change can also be used in combination. In that case, the results obtained at that wavelength can be taken as a base line, making it possible to carry out continuous measurement without separately determining a base line. Such measurement using a plurality of wavelengths may be embodied as follows.

Referring to Fig. 3, any adverse effects of, for example, possible fluctuation of the intensity of light from the light source may be reduced by comparing the difference in computer outputs of the reflectances at 520 nm (at this wavelength, HCl concentration does not influence the reflectance) and at 470 nm (at which the reflective spectrum shows its peak in the presence of HCl). Further, in Fig. 4, while the change in the output of reflectance measured at 470 nm is about 20%, the sum of the changes in reflectance at 430 nm and 470 nm gives a total difference of 35%. Measurement at a plurality of wavelengths is thus advantageous.

It should be noted that the wavelength at which a detector of the invention shows the spectrum peak can vary depending on the gas species with which said detector reacts. For example, although both Figs. 3 and 4 show the spectrum of EC/ZnBr4-TPP, differences are found between the spectra in the detection of hydrogen chloride (Fig. 3) and that of chlorine (Fig. 4). For example, when the reflectance is measured at 430 nm, 470 nm and 570 nm, the detector shows its peak only at 470 nm after reaction with hydrogen chloride, whereas the detector having reacted with chlorine shows a peak at each of the three wavelengths. Accordingly, it is possible to identify the gas species from the spectrum pattern.

Fig. 5 shows the response of EC/ZnBr4-TPP measured at wavelengths of 480 nm and 720 nm upon contact with nitrogen gas as background and nitrogen gas containing 5 ppm of HCl alternately. At first, nitrogen was made to flow, and at 0 sec an HCl-containing gas was flown. The output of reflectance fell to 77% and 93% when measured at 480 nm and 720 nm, respectively. At 200 sec, the gas was changed to neat nitrogen, whereupon the output returned to the base line, 100%, at each of the wavelengths. It is seen that reversible output characteristics are obtained with very good reproducibility at either wavelength and the response is quick. Thus, the pigments of the invention showing such reversibility not only provides a gas detector but also provides a means for enabling continuous detection of harmful gases in a gas mixture being discharged from a factory, or even a means for gas identification.

Fig. 6 shows the output change of a cobalt complex of TPP (hereinafter referred to as Co-TPP) in detection of hydrogen chloride gas (5 ppm or 0.5 ppm) in nitrogen gas, in which the change in light reflectance is plotted as a function of wavelength.

In the present invention at least one of the above mentioned harmful gases can also be detected by bringing a gas mixture to be detected into contact with a gas detector having a gas reactive pigment comprising TPP represented by formula (III) to induce a color change, irradiating the detector with light, and measuring the color change of the gas reactive pigment in terms of change in light absorption by using transmitted light or reflected light.

Tetraphenylporphyrin of formula (III) has been well known with its abbreviation, TPP. It has also been known that TPP can be used as a gas detector which undergoes certain changes upon contact with a specific gas. Moreover, in the known method the changes are measured by way of fluorescence. However, such a detection system lacks sensitivity. By contrast, in accordance with the present invention, gas detection using a TPP-containing detector can be carried out at satisfactory sensitivity by irradiating the detector with light, and measuring the color change which occurs upon contact with a harmful gas by way of the change of absorption spectrum in the transmitted or reflected light from the detector.

The present invention will now be illustrated in greater detail, but it is to be understood that the present invention is not limited to the following embodiments.

The gas reactive pigments used in these examples can be obtained as follows:
1. TPP, CoTPP, ZnTPP and FeTPPCl --- (III) (I)
   These compounds can be prepared in accordance with the method described in Reference 1. TPP is commercially available.
2. Br4-TPP --- (II)
   This compound can be prepared in accordance with the method described in Reference 2 or 3.
3. CoBr4-TPP, ZnBr4-TPP and FeBr4-TPP --- (I)
   These compounds can be synthesized in accordance with the methods described in Reference 1, 2 or 4.
4. Br8-TPP
   This compound can be synthesized in accordance with the method described in Reference 4.
5. CoBr8-TPP, ZnBr8-TPP --- (I)
   These compounds can be synthesized in accordance with the method described in Reference 4, 5 or 6.
6. 5,10,15,20-tetrakis(4-hydroxyphenyl)-21H,23H-porphyrin (TP(OH)P); 5,10,15,20-tetrakis(4-methoxypheynyl)-21H,23H-porphyrin (TP(OCH3)P); 5,10,15,20-tetrakis(4-bromophenyl)-21H,23H-porphyrin (TP(Br)P); and tetrakis(4-carboxyphenyl)porphyrin (TCPP) are commercially available from Tokyo Kasei Kogyo Co., LTd., Tokyo Japan.

### References

1. Fuhrhop J. H. Smith K. M. in Porphyrins and metalloporhyrins; Smith K. M. ed; Elsevier; New York, 1975 Chapter 19
2. Giraudeau, A., Callot, H. J., Jordan J., Ezaher I., Gross, M., J. Am. Chem. Soc. 1979, 101, 3857
3. Callot H., J. Bull. Soc. Chim. France 1974, 7-8, 1492
4. Giraudeau. A., Callot, H. J., Gross, M., Inorg. Chem., 1979, 18, 201
5. Byrappa, P., Krrshnan, V., Inorg. Chem., 1991, 30, 239
6. D'Souza, Villard A., Van Caemelbecke E., Franzen, M., Boschi T., Tagliatesta P., Kadish K. M., Inorg. Chem., 1993, 32, 4042

### Example 1

Reactive pigments comprising a zinc complex of TPP or a derivative thereof shown in Table 1 below were prepared. Each reactive pigment was dissolved in a solvent together with ethyl cellulose, and the solution was applied to optical fiber 12 to prepare a detector. The resulting detector was set in measuring cell 11 of the detection apparatus of Fig. 2.

Nitrogen gas containing 5 ppm of hydrogen chloride or 0.5 ppm of chlorine (gas 4 to be examined) was introduced through passage 13 of measuring cell 11.

The results obtained are shown in Table 1. In Table 1, EC/ denotes that the detector comprises the reactive pigment together with ethyl cellulose polymer; Zn represents transition metal zinc; TPP represents tetraphenylporphyrin; and the numerals in Br4 and Br8 represent the numbers of bromine atoms bonded to the porphyrin skeleton.

**Table 1**

| Zn containing pigment | | | | | |
|---|---|---|---|---|---|
| Detector | | 5 ppm HCl | | 0.5 ppm Cl₂ | |
| No. | Structure | Sensitivity¹⁾ (Output Change) (%) | Reversibility) | Sensitivity¹⁾ (Output Change) (%) | Reversibility |
| 1 | EC/Zn-TPP | +²⁾ | - | 10 | slightly reversible |
| 2 | EC/ZnBr4-TPP | 25 | reversible | 18 | slightly reversible |
| 3 | EC/ZnBr8-TPP | 4 | reversible | 25 | irreversible |

| | | | | | |
|---|---|---|---|---|---|
| Note: 1) The percent output change at the main peak. | | | | | |
| 2) The change is visually detectable but not measurable quantitatively (hereinafter the same). | | | | | |

Detector No. 1 (EC/Zn-TPP) shows an output change of 10% on contact with 0.5 ppm chlorine, which change was slightly reversible. Detector No. 2 (EC/ZnBr4-TPP) exhibits high sensitivity to 5 ppm hydrogen chloride, showing a 25% output change with reversibility. It also shows a 18% output change to 0.5 ppm chlorine, which change was slightly reversible.

Detector No. 3 (EC/ZnBr8-TPP) shows an output change of 25% to 0.5 ppm chlorine, although the change was irreversible.

In the detection of chlorine gas, while the reflectance change as measured at 460 nm is about 20% (see Table 1 and Fig. 4), the sum of the differences of reflectance measured at 460 nm and 430 nm amounts to 35%. Therefore, chlorine gas can be measured at an increased sensitivity. Thus, measurement of the color change using a plurality of wavelengths is a preferred method.

### Example 2

A reactive pigment comprising an iron complex of a TPP derivative shown in Table 2 below was prepared. The reactive pigment was dissolved in a solvent together with ethyl cellulose, and the solution was applied to optical fiber 12 to prepare a detector. The resulting detector was set in the measuring cell 11 of the apparatus of Fig. 2.

Hydrogen chloride was detected using the detector in the same manner as in Example 1. The results obtained are shown in Table 2.

**Table 2**

| Fe containing pigment | | | |
|---|---|---|---|
| Detector No. | Detector Structure | 1 ppm HCl | |
| | | Sensitivity (Output Change) (%) | Reversibility |
| 4 | EC/FeBr8-TPP | 10 | reversible |

The results in Table 2 prove that a 10% output change can be obtained when the number of bromine atoms in the TPP derivative complex is 8. It seems that the pigments containing 0 to 4 bromine atoms do not have a practical level of sensitivity.

### Example 3

Reactive pigments comprising the iron complexed of TPP derivative shown in Table 3 below were prepared. Each of the reactive pigments was dissolved in a solvent together with ethyl cellulose, and the solution was applied to optical fiber 12 to prepare a detector. The resulting detector was set in measuring cell 11 of the detection apparatus of Fig. 2, and measurement was made in the same manner as in Example 1. The results obtained are shown in Table 3.

**Table 3**

| Pigment containing no metal | | | | | |
|---|---|---|---|---|---|
| Detector | | 1 ppm HCl | | 10 ppm NOₓ | |
| No. | Structure | Sensitivity (Output Change) (%) | Reversibility | Sensitivity (Output Change) (%) | Reversibility |
| 5 | EC/Br4-TPP | 18 | reversible | 35 | reversible |
| 6 | EC/Br8-TPP | 15 | reversible | 30 | reversible |

Detector No. 5 (EC/Br4-TPP) shows a reversible output change of 18% for hydrogen chloride (1 ppm). Detector No. 6 (EC/Br8-TPP) shows a reversible output change of 15% for hydrogen chloride (1 ppm).

Both detector Nos. 5 and 6 exhibit reversibility in their change on contact with NOₓ, having a sensitivity of about 30 to 40%.

### Example 4

In order to observe the effects of Ethyl Violet used in combination with the reactive pigment of the invention, detectors were prepared from the reactive pigment comprising the TPP metal complex (Zn-TPP). The pigment singly or in combination with Ethyl Violet was dissolved in a solvent together with ethyl cellulose and the solution was applied to optical fiber 12. The detector was placed in measuring cell 11 of the detection apparatus of Fig. 2, and measurement was made in the same manner as in Example 1. The results obtained are shown in Table 4. In Table 4, EV stands for Ethyl Violet, and EC-EV/Zn-TPP refers to the detector containing Ethyl Violet.

**Table 4**

| Combined Use of Ethyl Violet | | | | | |
|---|---|---|---|---|---|
| Detector | | 5 ppm HCl | | 0.5 ppm Cl₂ | |
| No. | Structure | Sensitivity (Output Change) (%) | Reversibility | Sensitivity (Output Change) (%) | Reversibility |
| 7 | EC/Zn-TPP | + | - | 10 | slightly reversible |
| 8 | EC-EV/Zn-TPP | 12 | slightly reversible | 13 | slightly reversible |

As can be seen from Table 4, a combined use of Ethyl Violet brings about further improvement in sensitivity.

### Example 5

A gas reactive pigment comprising the TPP metal complex containing cobalt as the chelating metal as shown in Table 5 was prepared. The gas reactive pigment was dissolved in a solvent together with ethyl cellulose, and the solution was applied to optical fiber 12 to prepare a detector. The detector was set in measuring cell 11 of the apparatus shown in Fig. 2.

As gas to be detected, nitrogen gas containing 5 ppm of hydrogen chloride or 0.5 ppm of chlorine was introduced through passage 13 of measuring cell 11, and measurement was made in the same manner as in Example 1. The results obtained are shown in Table 5.

**Table 5**

| Co containing pigment | | | | | |
|---|---|---|---|---|---|
| Detector | | 5 ppm HCl | | 0.5 ppm Cl₂ | |
| No. | Structure | Sensitivity (Output Change) (%) | Reversibility | Sensitivity (Output Change) (%) | Reversibility |
| 9 | EC/Co-TPP | 9 | irreversible | 17 | irreversible |

It is seen from the results that detector No. 9, EC/Co-TPP, shows a 9% output change on contact with 5 ppm hydrogen chloride. No reversibility was observed in that change. On contact with 0.5 ppm chlorine gas a 17% output change was observed, which change was not reversible.

### Example 6

A solution of a gas reactive pigment and ethyl cellulose in a solvent was applied to alumina or glass substrate 2 to prepare detector 3. The detector 3 was set in measuring cell 1 as shown in Fig. 1. Projector/receptor element 7 was provided in front of the substrate 2 so that the light from halogen lamp 6 was led to the projector of element 1) through an optical fiber so that, emitted from the projector, reflected on detector 3, and received by the receptor of said element 7. The signals from the receptor were sent to multiphotometry detection instrument 8. Gas 4 to be examined was introduced into measuring cell 1, and the color change of the reactive pigment on reaction was measured with multiphotometry detection instrument 8. The results of measurement were processed by computer 9.

In Examples 7 to 12, the measurements were conducted as described in this example.

### Example 7

### Reactive Pigment (II) (G=Br):

Gas reactive pigment TP(Br)P and ethyl cellulose were dissolved in a common solvent, and the solution was applied to an alumina substrate to prepare a detector.

The detector was placed in the detection apparatus of Fig. 1, and nitrogen gas containing 0 to 5 ppm of hydrogen chloride was examined.

The spectrum change of the detector is shown in Fig. 7. The reflectance showed an increase when measured at 420 nm and 520 nm and a decrease when measured at 455 nm and 670 nm. These output changes were reversible.

Fig. 8 shows the relationship of log(100/reflectance) vs. hydrogen chloride gas concentration at 453 nm. Substantial changes in output were observed at a hydrogen chloride gas concentration of from 0.2 to 3 ppm, and the change reached saturation at 3 ppm or higher.

It is thus seen that the gas reactive pigment TP(Br)P has a broader detectable range than TPP. It is possible to detect hydrogen chloride gas in concentrations in the order of several ppm by monitoring any of the peaks at the above-described wavelengths.

### Example 8

### Reactive Pigment (II) (G=OH):

Gas reactive pigment TP(OH)P and ethyl cellulose were dissolved in a common solvent, and the solution was applied to an alumina substrate to prepare a detector.

The detector was placed in the detection apparatus of Fig. 1, and nitrogen gas containing 0.2 ppm of hydrogen chloride was examined.

The change in spectrum of the detector is shown in Fig. 9. The reflectance showed an increase when measured at 425 nm, 520 nm, and 560 nm, and a decrease when measured at 450 nm and 710 nm. These output changes were reversible but required time for restoration.

Fig. 10 shows the change of log(100/reflectance) vs. time in the reaction with 0.2 ppm of hydrogen chloride, as measured at 450 nm, 520 nm and 710 nm. Upon exposure to 0.2 ppm hydrogen chloride gas, the change reached saturation in 1000 seconds.

The results indicates that the detector using TP(OH)P responds to even a low concentration of hydrogen chloride gas if exposed thereto for an extended period of time, namely, showing cumulative responses.

### Example 9

### Reactive Pigment (II) (G=OCH₃):

Gas reactive pigment TP(OCH₃)P and ethyl cellulose were dissolved in a common solvent, and the solution was applied to an alumina substrate to prepare a detector.

The detector was placed in the detection apparatus of Fig. 1, and nitrogen gas containing 0 to 5 ppm of hydrogen chloride was examined.

Fig. 11 shows the spectrum change of the detector. The reflectance increased at 420 nm and decreased at 455 nm and 700 nm. These changes were reversible. The output began to change at a hydrogen chloride gas concentration of about 0.2 ppm, and the change reached saturation at 1 ppm or more.

It is thus possible to detect hydrogen chloride gas in concentrations in the order of several ppm by monitoring the peaks at the above-described wavelengths.

Fig. 12 shows the relationship of log(100/reflectance) vs. hydrogen chloride gas concentration at 457 nm. It can be seen that the output begins to change at hydrogen chloride concentrations of about 0.2 ppm, and the change reaches saturation at 1 ppm or more.

### Example 10

### Reactive Pigment (II) (G=COOH):

Gas reactive pigment TP(COOH)P and ethyl cellulose were mixed and dissolved in a solvent, and the solution was applied to an alumina pigment to prepare a detector.

The detector was placed in the detection apparatus of Fig. 1, and nitrogen gas containing 0 to 9 ppm of hydrogen chloride was examined.

Fig. 13 shows the spectrum change of the detector. It can be seen that the reflectance decreased at 458 nm. This change was reversible.

Fig. 14 shows a relationship of log(100/reflectance) vs. hydrogen chloride gas concentration at 458 nm. It is seen that the output changes in a dose dependent manner at hydrogen chloride concentrations between 1 to 9 ppm or more. That is, the range of hydrogen chloride gas concentration detectable by the output change is broader than those of the foregoing examples.

It is thus possible to detect hydrogen chloride gas in concentrations in the order of several ppm by monitoring the peak at the above-described wavelength.

### Example 11

### Reactive Pigment (II) (G=SO₃H):

A detector was prepared using a gas reactive pigment, TP(SO₃H)P, in the same manner as in Example 10. When used for detection of ammonia gas (equilibrated steam from 25% aqueous ammonia was used), the detector turned from green or yellowish green to brown. It was thus confirmed that the gas reactive pigment is responsive to ammonia.

### Example 12

### Reactive Pigment (II) (G=Br):

A detector was prepared using the gas reactive pigment, TP(Br)P, in the same manner as in Example 10. When used for detection of HF gas or formic acid gas (equilibrated steam from hydrofluoric acid or formic acid was used), the detector turned from green or yellowish green to brown. It was thus confirmed that the gas reactive pigment is responsive to these gases.

### Example 13

TPP of formula (III) was used as a gas reactive pigment, which was dissolved together with ethyl cellulose in a common solvent, and the solution was applied to optical fiber 12 to prepare a detector. The detector was placed in measuring cell 11 of the detection apparatus of Fig. 2, and measurement was performed in the same manner as in Example 10, except that the intensity of transmitted light was measured. The results obtained are shown in Table 6 below.

**Table 6**

| Transmission Measurement using TPP | | | | | |
|---|---|---|---|---|---|
| Detector | | 1 ppm HCl | | 10 ppm NOₓ | |
| No. | Structure | Sensitivity (Output Change) (%) | Reversibility | Sensitivity (Output Change) (%) | Reversibility |
| 10 | EC/TPP | 40 | reversible | 40 | reversible |

As has been fully described, the gas reactive pigments of the invention are highly sensitive to at least one of a halogen gas, a hydrogen halide gas, an acidic gas, an oxidizing gas, a basic gas, and an organic acid gas, preferably a halogen gas and a hydrogen halide gas, and particularly chlorine gas and hydrogen chloride gas, which may exist even at a low concentration. The pigments show noticeable changes in color or spectrum on contact with these gases, and therefore, it is possible to use them to detect these harmful gases at high sensitivity. Some of the gas reactive pigments of the invention can be used for continuous measurement with high detection sensitivity and satisfactory durability for repeated use.

The detectors, detecting methods, and detection apparatus of the invention are effective for detecting any accidental failure of the adsorbent used in an exhaust gas treatment equipment, or for gas leakage detecting from such equipment, especially in semiconductor production systems.

While the invention has been described in detail and with reference to specific embodiments thereof, it will be apparent to one skilled in the art that various changes and modifications can be made without departing from the spirit and scope of the invention.

## Claims

1. A gas reactive pigment comprising a metal complex of tetraphenylporphyrin or a derivative thereof represented by formula (I): 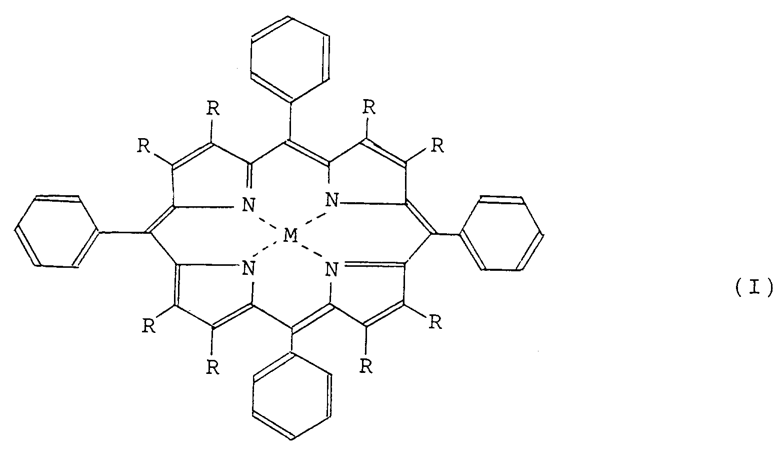 wherein M represents a transition metal; a plurality of R's independently represent a hydrogen atom, a halogen atom, a nitro group, a cyano group or an alkoxy group, said pigment having reactivity to at least one of a halogen gas, a hydrogen halide gas, an acidic gas, an oxidizing gas, a basic gas, and an organic acid gas.

2. A gas reactive pigment according to claim 1, wherein at least one of the plurality of R's in formula (I) is a bromine atom.

3. A gas reactive pigment according to claim 1, wherein M in formula (I) is zinc.

4. A gas reactive pigment according to claim 1, wherein in formula (I) M is zinc, and at least one of the plurality of R's is a bromine atom.

5. A gas reactive pigment according to claim 1, wherein in formula (I) M is zinc, and 3 to 5 out of the plurality of R's are a bromine atom.

6. A gas reactive pigment according to claim 1, wherein in formula (I) M is iron, and all of the plurality of R's are a bromine atom.

7. A gas reactive pigment comprising a tetraphenylporphyrin derivative represented by formula (II): 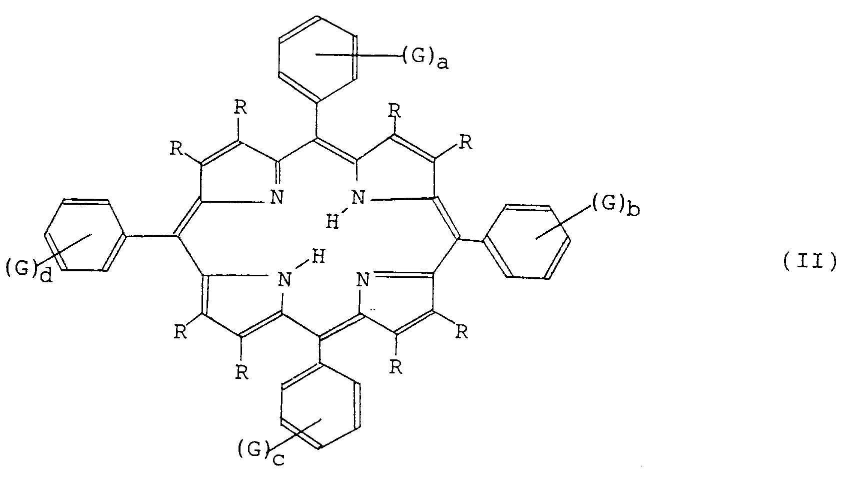 wherein a plurality of R's independently represent a hydrogen atom, a halogen atom, a nitro group, a cyano group or an alkoxy group; a plurality of G's on the 4 phenyl groups all represent a substituent selected from a halogen atom, a hydroxyl group, a sulfonic acid group, a carboxyl group, an alkoxy group, and an amino group, the positions of which on the 4 phenyl groups may be the same or different; and a, b, c, and d, which may be the same or different, each represents an integer of 0 to 5, indicating the number of G's on each phenyl group; provided that the compound wherein all R's represent a hydrogen atom, and a, b, c, and d all represent 0 is excluded,
said pigment having reactivity to at least one of a halogen gas, a hydrogen halide gas, an acidic gas, an oxidizing gas, a basic gas, and an organic acid gas.

8. A gas reactive pigment according to claim 7, wherein G in formula (II) is a hydroxyl group, a carboxyl group, a sulfonic acid group, a bromine atom or a methoxy group.

9. A gas detector for detecting at least one of a halogen gas, a hydrogen halide gas, an acidic gas, an oxidizing gas, a basic gas, and an organic acid gas, which comprises a gas reactive pigment claimed in any of claims 1 to 8.

10. A gas detector according to claim 9, wherein said gas reactive pigment is supported on a carrier.

11. A gas detector according to claim 10, wherein said detector is prepared by the steps of dissolving the gas reactive pigment in a solvent together with a matrix polymer, applying the solution to the surface of a carrier, and drying the solution to remove the solvent.

12. A gas detector according to claim 11, wherein said matrix polymer is a polymer soluble in a solvent for said gas reactive pigment, and said carrier is an alumina substrate, a glass substrate, an optical fiber, paper, glass beads, or silica gel.

13. A gas detector according to claim 9, wherein said detector further comprises Ethyl Violet in addition to said gas reactive pigment.

14. A method for detecting at least one of a halogen gas, a hydrogen halide gas, an acidic gas, an oxidizing gas, a basic gas, and an organic acid gas, which comprises the steps of:
bringing a gas reactive pigment as claimed in any of claims 1 to 8 or a gas detector as claimed in any of claims 9 to 13 into contact with gas to be detected, and qualitatively or quantitatively measuring any change of the color occurring in the gas reactive pigment upon said contact.

15. A method according to claim 14, wherein the color change of the gas reactive pigment is measured on the basis of the change taking place in the absorption or reflective spectrum of the gas reactive pigment, and the measurement is performed in the light which is transmitted through or reflected from said detector.

16. A method according to claim 15, wherein the measurement is performed continuously while said detector is being irradiated.

17. A method according to claim 15 or 16, wherein the change in the absorption or reflective spectrum of the gas reactive pigment is measured on the basis of the changes in absorbancy at a plurality of wavelengths.

18. A method according to claim 17, wherein the difference in color changes at the plurality of wavelengths, serves to enhance the sensitivity in the gas detection.

19. A method for detecting at least one of a halogen gas, a hydrogen halide gas, an acidic gas, an oxidizing gas, a basic gas, and an organic acid gas, which comprises the steps of:
bringing gas to be detected into contact with a gas reactive pigment comprising tetraphenylporphyrin represented by formula (III) or a gas detector containing said pigment: 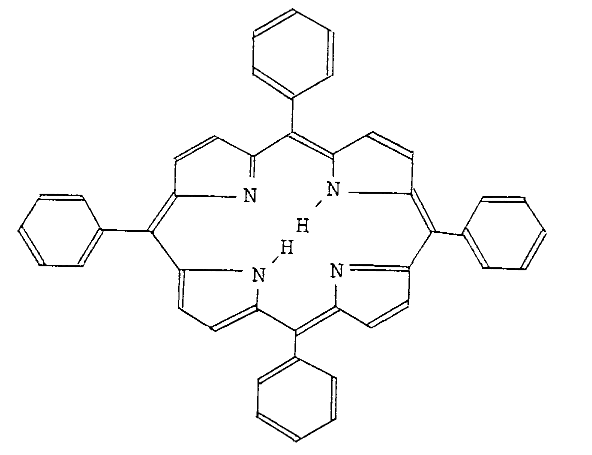 irradiating the detector with light, and measuring any change of the color of the gas reactive pigment in terms of the change in light absorption of the gas reactive pigment by using transmitted or reflected light from the detector.

20. A gas detection apparatus for detecting at least one of a halogen gas, a hydrogen halide gas, an acidic gas, an oxidizing gas, a basic gas, and an organic acid gas, which has a gas detector claimed in any of claims 9 to 13 in the passage of gas to be examined.
